Europäisches Patentamt

European Patent Office

Office européen des brevets

(19)

(11) Publication number: **0 148 552**
**A1**

# (12) EUROPEAN PATENT APPLICATION

(21) Application number: **84303755.7**

(22) Date of filing: **04.06.84**

(51) Int. Cl.⁴: **C 12 N 15/00**
**// C12R1/465**

(30) Priority: **07.06.83 GB 8315579**

(43) Date of publication of application: **17.07.85**
**Bulletin 85/29**

(84) Designated Contracting States: **AT BE CH DE FR GB IT LI LU NL SE**

(71) Applicant: **BIOGEN N.V., 15 Pietermaai, Willemstad Curacao, Netherlands Antilles (NL)**

(72) Inventor: **Thompson, Charles J., 19 Chemin des Palettes, Ch-1212 Grand-Lancy (CH)**
Inventor: **Gray, Gary S., 6 Southerland Road, Apt. 33, Brookline Massachusetts 02146 (US)**

(74) Representative: **Bannerman, David Gardner et al, Withers & Rogers 4 Dyer's Buildings Holborn, London, EC1N 2JT (GB)**

(54) Streptomyces expression sequence and methods for expressing DNA sequences in streptomyces.

(57) Streptomyces expression systems and methods for expressing foreign DNA sequences in Streptomyces characterized by at least one promoter selected from the group consisting of the promoters of Streptomyces aminoglycoside phosphotransferases. These expression systems and methods enable the expression of eukaryotic and viral DNA sequences in Streptomyces and the production of various pharmaceutical, veterinary and industrial products in those hosts.

EP 0 148 552 A1

ACTORUM AG

## STREPTOMYCES EXPRESSION SYSTEMS AND METHODS
## FOR EXPRESSING DNA SEQUENCES IN STREPTOMYCES

### TECHNICAL FIELD OF INVENTION

This invention relates to Streptomyces expression systems and to methods of expressing foreign DNA sequences in Streptomyces. More particularly, the invention relates to methods for producing polypeptides and proteins coded for by those foreign DNA sequences in Streptomyces. These polypeptides and proteins are then useful in vaccines, in pharmaceutical and animal health care products, in industrial products, and as intermediates in the production of those products or other such products by more traditional chemical or biological processes.

### BACKGROUND ART

Numerous eukaryotic and viral genes have been expressed in the gram-negative bacterium, Escherichia coli. Examples of such expression include the production of polypeptides displaying a biological or immunological activity of human leukocyte interferons [S. Nagata et al., "Synthesis In E. coli Of A Polypeptide With Human Leukocyte Interferon Activity", Nature, 284 pp. 316-20 (1980)], antigens displaying the specificity of human hepatitis B viral antigens [C. J. Burrell et al., "Expression In Escherichia coli Of Hepatitis B Virus DNA Sequences Cloned In Plasmid pBR322", Nature, 279, pp. 43-47 (1979);
M. Pasek et al., "Hepatitis B Virus Genes And Their
Expression In E. coli", Nature, 282, pp. 575-79
(1979)] and polypeptides displaying the antigenicity
of FMD viral antigens [H. Küpper et al., "Cloning
Of cDNA Of Major Antigen Of Foot And Mouth Disease
Virus And Expression In E. coli.", Nature, 289,
pp. 555-59 (1981)].

However, the production of such useful
eukaryotic and viral products in E. coli is characterized by several disadvantages. For example, because
E. coli are routinely present in the intestinal tract
of animals and humans, careful handling of E. coli
hosts that have been modified to produce a desired
product is required to prevent accidental infection.
At present, the E. coli hosts that are permitted to
be used to produce desired products are limited to
those that will not grow outside of the laboratory
environment. Moreover, the transformed E. coli hosts
must be grown in closed systems under high conditions
of containment (as now mandated by the guidelines
of the National Institute of Health, and other like
governmental organizations). The resultant E. coli
cultures must also be processed to kill the bacteria
before opening the system to the environment. These
requirements, obviously, greatly increase the material
and equipment costs of fermentation processes using
E. coli. In addition, some of the bacterial products
of E. coli are endotoxins. Therefore, before eukaryotic and viral products produced in E. coli hosts
are able to be be used in animals and humans, the
E. coli endotoxins must be totally eliminated from.
the desired product. Obviously, this elimination
also entails costly and cumbersome purification and
isolation procedures.

Numerous eukaryotic and viral genes have
also been expressed in various strains of the gram-

positive bacterium <u>Bacillus</u>.  Examples of such expression include the hepatitus B viral core antigens [K. Hardy et al, "Production In <u>B. subtilis</u> Of Hepatitis Core Antigen And Of Major Antigen Of Foot And Mouth Disease Virus", <u>Nature</u>, 293, pp. 48-P3 (1981)], and the antigens of foot and mouth disease virus [K. Hardy et al, <u>supra</u>].

The production of such products in <u>Bacillus</u> hosts is advantaged in many respects, as compared to <u>E. coli</u> hosts.  For example, the most commonly used <u>Bacillus</u> host, <u>B. subtilis</u>, is non-pathogenic. It does not have the capacity to infect humans or animals.  It also does not produce endotoxins.  Therefore, the production of eukaryotic and viral proteins in <u>B. subtilis</u> hosts avoids the disadvantageous high containment fermentation and harvesting conditions mandated for <u>E. coli</u> fermentations.  It also avoids the necessity of purifying the product to remove all traces of host-produced toxins.  Finally, <u>Bacillus</u> hosts naturally secrete several of their protein products into the extracellular space of the cell. Therefore, such products may be recovered readily from the cell and purified without the attendant contaminants derived from lysis or break-up of the host cells.

Although <u>E. coli</u> and <u>Bacillus</u> hosts are now being used to produce proteins and polypeptides encoded for by various foreign DNA sequences used to transform those hosts, the full potential of recombinant DNA technology has not been realized in these hosts.  There are several reasons for this failure.  For example, it is difficult to grow <u>E. coli</u> and <u>Bacillus</u> hosts to very high density.  Accordingly, fermentation yields are lower on a per-volume basis than would otherwise be possible with cultures able to be grown to higher density.  In addition, <u>E. coli</u> and <u>Bacillus</u> hosts do not remain active producers of the desired products over long periods of time unless they are immobilized. Again, this results in lower fermentation yields than would otherwise be obtainable.

These disadvantages of E. coli and Bacillus hosts may, however, be avoided by the use of Streptomyces hosts to produce the desired eukaryotic and viral products. Streptomyces are non-toxic. None is known to cause disease in, or be a commensal of, man or animals. Moreover, Streptomyces are widely employed in industrial fermentations to produce antibiotics such as aminoglycosides, macrolides and β-lactam, polyether and glycopeptide antibiotics. Accordingly, there is a wide range of knowledge about, and experience with Streptomyces fermentations and purifications available to the art. For example, Streptomyces may be grown to very high culture densities. Streptomyces also exhibit a secondary metabolism, after the completion of vegetative growth, that permits them to remain active synthesizers of a desired product for several weeks. Finally, Streptomyces excrete many of their products during this secondary metabolism. Because Streptomyces contain a wider variety of such extracellular hydrolytic enzymes, e.g., proteases, amylases, cellulases, lipases, nucleases and others, than E. coli or Bacillus, Streptomyces hosts are more adaptable to the excretion of desired products than the former two host genera.

However, in spite of the advantages of employing Streptomyces hosts in recombinant DNA technology to produce desired eukaryotic, viral and other foreign products, no successful cloning system for the expression of foreign DNA sequences, particularly eukaryotic or viral DNA sequences, in Streptomyces hosts has been reported.

0148552

Instead, the former work with Streptomyces has been limited to investigations of Streptomyces genetics, its plasmids, phages and DNA, methods for reintroducing Streptomyces DNA into Streptomyces, and the expression of bacterial-derived DNA sequences in those hosts. See, e.g., K. F. Chater et al., "Gene Cloning In Streptomyces", Current Topics in Microbiology and Immunology, pp. 69-85 (1982); C. J. Thompson et al., "Biochemical Characterization Of Resistance Determinants Cloned From Antibiotic-Producing Streptomycetes", J. Bact., 151, pp. 678-85 (1982); C. J. Thompson et al., "Cloning Of Antibiotic Resistance And Nutritional Genes In Streptomycetes", J. Bact., 151, pp. 668-77 (1982); C. J. Thompson et al., "Physical Analysis Of Antibiotic-Resistance Genes From Streptomyces And Their Use In Vector Construction", Gene, 20, pp. 51-62 (1982); T. Kieser et al., "pIJ101, A Multi-Copy Broad Host-Range Streptomyces Plasmid: Functional Analysis And Development Of DNA Cloning Vectors", Mol. Gen. Genet., 185, pp. 223-38 (1982); M. J. Bibb et al., "Development Of a Cloning System For Streptomyces", Dev. Ind. Microbiol., 21, pp. 55-64 (1980); C. J. Thompson et al., "DNA Cloning In Streptomyces: Resistance Genes From Antibiotic-Producing Species", Nature, 286, pp. 525-27 (1980); and European patent application 77,649.

None of these investigations has resulted in the cloning of a eukaryotic, viral or other foreign DNA sequence into a streptomycete or its expression in that host to produce the polypeptide coded for by it.

## SUMMARY OF THE INVENTION

The present invention generally solves the problems referred to above by, for the first time, providing Streptomyces expression systems and methods for using those systems to express foreign DNA sequences, particularly eukaryotic and viral DNA sequences, in Streptomyces. More particularly, we provide in accordance with this invention a Streptomyces expression system for expressing foreign DNA sequences in Streptomyces that is characterized by at least one promoter selected from the group consisting of the promoters of Streptomyces aminoglycoside phosphotransferases.

By virtue of our invention, it is possible for the first time to employ Streptomyces hosts to synthesize eukaryotic, viral and other foreign polypeptides and proteins in Streptomyces. Moreover, the expression systems of our invention permit the cloning of the foreign DNA sequences coding for those desired products, the transformation of Streptomyces hosts with those DNA sequences, and the expression of those foreign DNA sequences in Streptomyces. Accordingly, the expression systems and methods of this invention for the first time enable the advantages of Streptomyces hosts to be realized in the production and excretion of products through recombinant DNA technology.

As will be appreciated from the disclosure to follow, this novel combination of recombinant DNA technology and Streptomyces hosts enables the large scale and high yield production of products useful in a wide variety of pharmaceutical, animal health and industrial applications.

The promoters and expression systems of this invention may also usefully be employed to amplify the expression of Streptomyces-derived DNA sequences that encode various antibiotics and other useful products.

B.0217

## BRIEF DESCRIPTION OF THE DRAWINGS

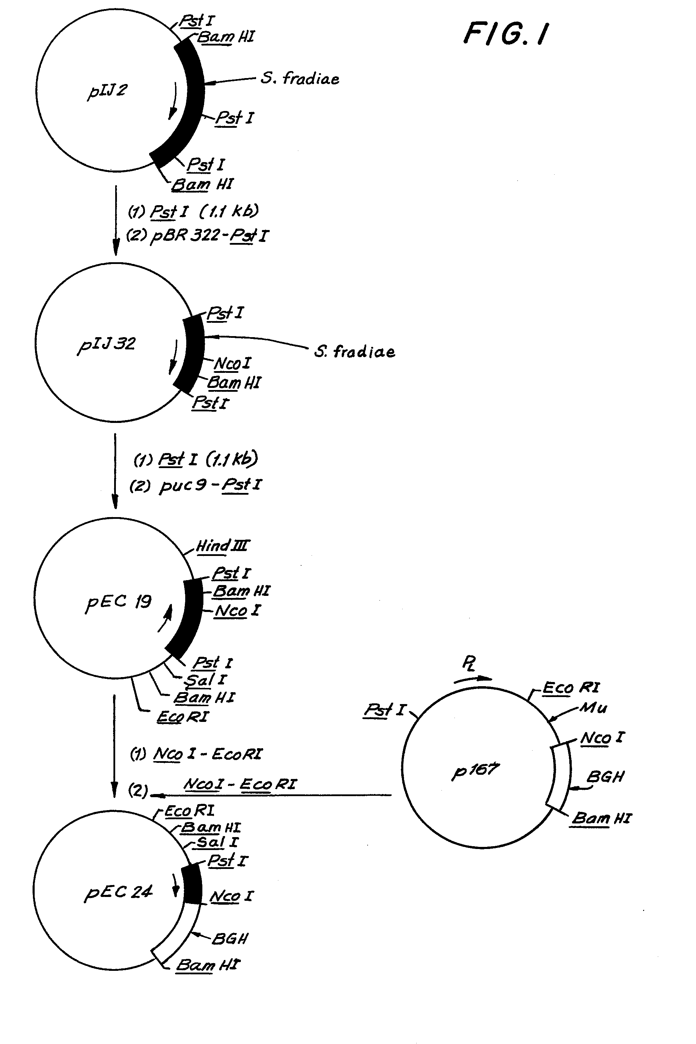

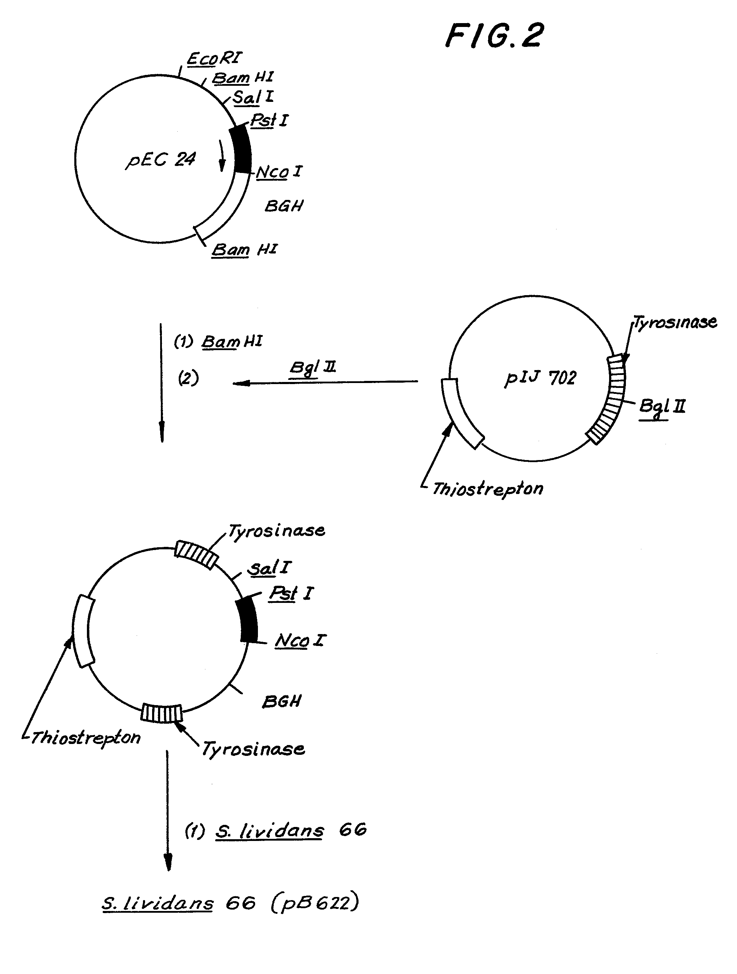

Figures 1 and 2 are a schematic outline of one embodiment of a <u>Streptomyces</u> expression system of this invention and of a method of employing that system to produce a desired animal hormone (bovine growth hormone).

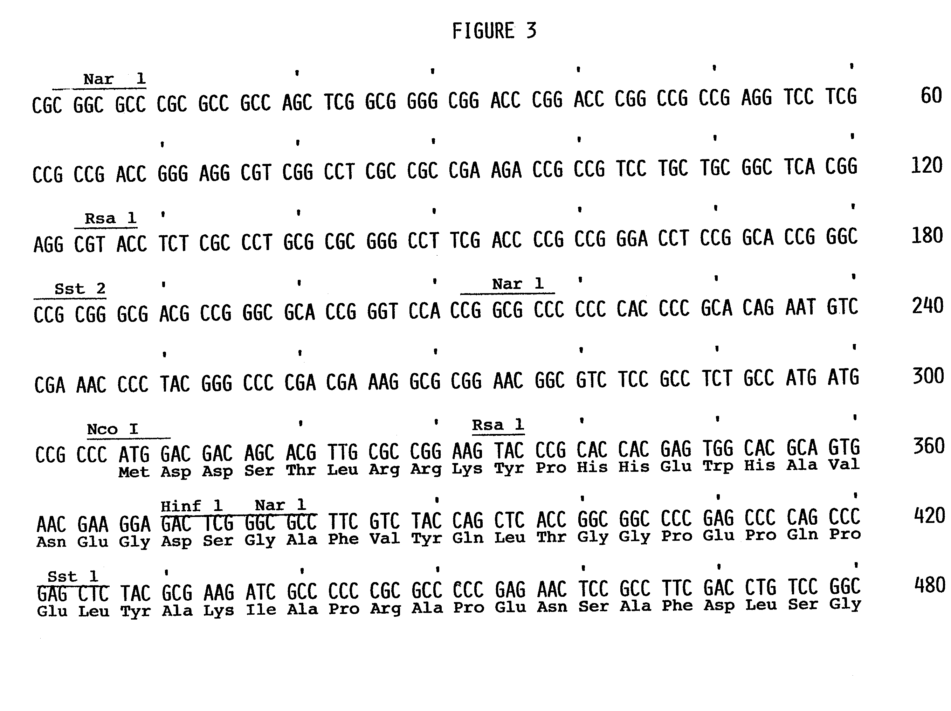

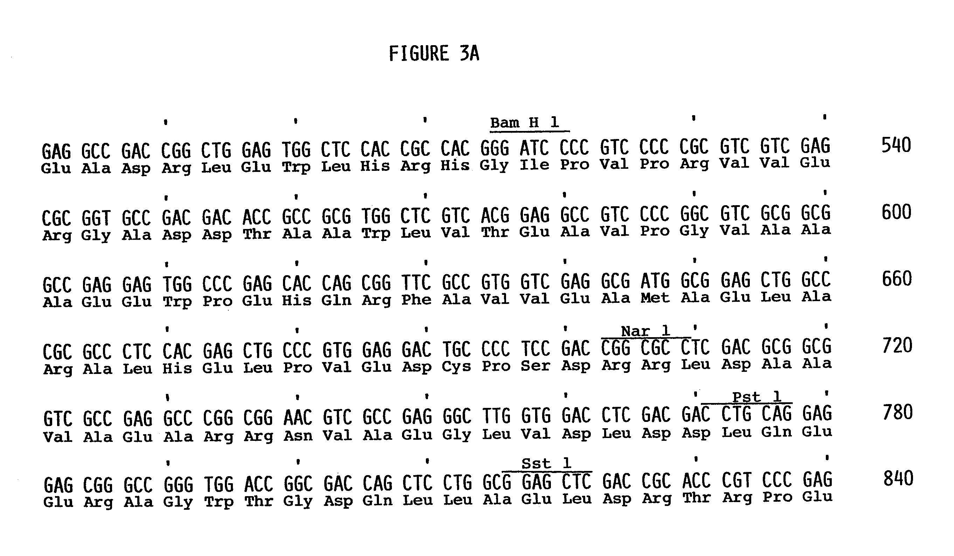

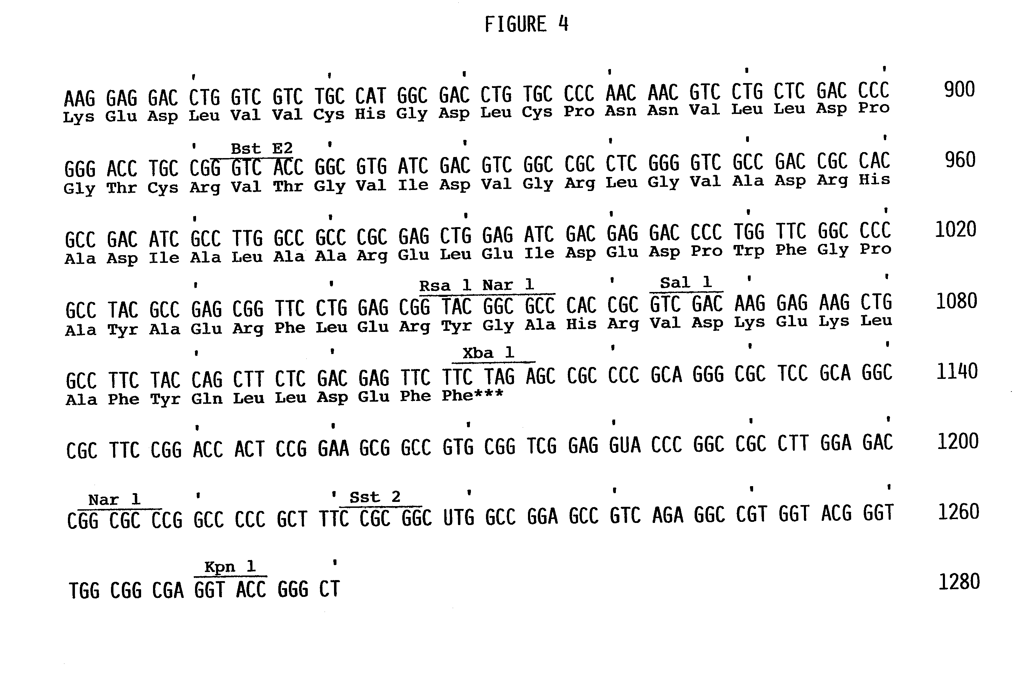

Figures 3 and 4 depict the nucleotide sequence encoding and the amino acid sequence of neomycin phosphotransferase and portions of the 3' and 5' non-coding regions of that DNA sequence. The promoter and other expression control sequences of the neomycin phosphotransferase coding sequence are located in the 5' non-coding region depicted in Figure 3 between the <u>Sst</u>II site and the start of the coding region.

## BEST MODE OF CARRYING OUT THE INVENTION

In order that this invention may be more fully understood, the following detailed description is set forth. In this description the following terms are employed:

<u>Polypeptide</u> -- A linear array of amino acids connected one to the other by peptide bonds between the α-amino and carboxy groups of adjacent amino acids.

<u>Expression</u> -- The process undergone by a DNA sequence or gene to produce a polypeptide. It is a combination of transcription (the process of producing mRNA from a DNA sequence) and translation (the process of producing a polypeptide from mRNA).

<u>Plasmid</u> -- A non-chromosomal, double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance ($Tet^R$) transforms a cell previously sensitive to tetracycline into one which is resistant to it. A cell transformed by a plasmid is called a "transformant".

Phage or Bacteriophage -- Bacterial virus, many of which consist of DNA sequences encapsidated in a protein envelope or coat ("capsid protein").

Streptomyces Cloning Vector -- A plasmid, phage DNA or other DNA sequence that has (a) an intact replicon, such that the cloning vehicle is able to replicate in a Streptomyces host and (b) one or a small number of endonuclease recognition sites at which such DNA sequences may be cut in a determinable fashion without attendant loss of an essential biological function of the cloning vehicle, e.g., replication or production of coat proteins. Preferably, a Streptomyces cloning vector, as originally constructed, or after a foreign DNA sequence is inserted therein, also has a marker suitable for use in the identification of correctly transformed cells, e.g., viomycin resistance, thiostrepton resistance, erythromycin resistance, or the production of some other detectable protein product or sensitivity to such product or antibiotic. A Streptomyces cloning vector is often called a Streptomyces cloning vehicle.

Cloning -- The process of obtaining a population of organisms or DNA sequences derived from one such organism or sequence by asexual reproduction.

Recombinant DNA Molecule or Hybrid DNA -- A molecule consisting of segments of DNA from different genomes which have been joined end-to-end outside of living cells and have the capacity to infect some host cell and be maintained therein.

Streptomyces Expression Control Sequence -- A sequence of nucleotides that controls and regulates the expression of DNA sequences in a Streptomyces host, when operatively linked to those DNA sequences.*
A Streptomyces expression control sequence includes
a promoter region and the various operator regions,
ribosome binding sites, and other sequences, including,
if necessary, an ATG start signal to control and to
regulate the expression of a DNA sequence operatively
linked thereto.

Streptomyces Host -- A microbial strain
selected from the genus Streptomyces.  It may include, for example, S. acrimycini, S. albus,
S. azureus, S. fradiae, S. glaucescens, S. griseus,
S. parvulus, S. pristinaespiralis, S. rimosus,
S. violaceotuber, S. lividans, S. coelicolor and
other strains of the genus Streptomyces, such as
those listed in European patent application 77,649.

Foreign DNA Sequence -- A non-Streptomyces-
derived DNA sequence encoding a protein or polypeptide, particularly one of eukaryotic or viral origin.

As described previously, the Streptomyces
expression systems and methods of this invention
are characterized by at least one promoter selected
from the group consisting of the promoters of
Streptomyces aminoglycoside phosphotransferase.  A
large number of such promoters are known.  They are
believed to contribute to the resistance of a particular Streptomyces to the aminoglycoside made in
that streptomycete.  Examples of such aminoglycoside
phosphotransferases are streptomycin phosphotransferase, neomycin phosphotransferase and kanamycin

---

* The term "operatively linked" includes having
an appropriate start signal in front of the foreign
gene or DNA sequence encoding the desired product
and maintaining the correct reading frame to permit
expression of the foreign DNA sequence and production
of the desired product under the control of the expression control sequence.

phosphotransferase. We prefer neomycin and kanamycin phosphotransferase.

We have also found that when such promoters are employed in Streptomyces that do not produce any aminoglycoside, the activity of the promoter is greatly amplified, perhaps as a result of the absence of repressors in those non-aminoglycoside producing strains. Accordingly, we prefer to use such non-aminoglycoside producing strains as our Streptomyces host. However, the promoters of our invention are also active, although perhaps to a lesser extent, in aminoglycoside-producing strains of Streptomyces and those hosts may also be used in this invention.

In the expression systems and methods of this invention, the promoters of the various Streptomyces aminoglycoside phosphotransferases, described above, are employed in Streptomyces expression control sequences in such a way as to control and to regulate the expression of foreign DNA sequences operatively-linked to those expression control sequences in Streptomyces cloning vectors containing those expression control sequences and foreign DNA sequences. These vectors are then employed to transform Streptomyces hosts and the transformed hosts cultured to produce the desired polypeptides and proteins.

As we have previously described, these expression systems and methods are useful in the expression of various foreign DNA sequences, particularly those of eukaryotic and viral origin, in Streptomyces hosts. Examples of such eukaryotic and viral DNA sequences are sequences encoding human and animal leukocyte interferons (IFN-α), fibroblast interferons (IFN-β), and immune interferons (IFN-γ), human insulin, human and animal growth and other hormones, human serum albumin and various human blood factors and plasminogen activators, both tissue and urokinase, hepatitis B viral core and surface antigens, FMD viral antigens and other human, animal and viral polypeptides and proteins.

It should also be understood that in one embodiment of this invention, the _Streptomyces_ promoters and expression systems of this invention are employed to amplify the expression of _Streptomyces_-derived DNA sequences that code the various antibodies and other products. In this embodiment, the promoters and expression systems of this invention are employed to improve the yield or speed of any rate-limiting step in the biological pathway of antibiotic or other productions in _Streptomyces_. In such embodiment, the promoters or expression systems of this invention are operatively linked to the DNA sequence encoding the products of that rate-limiting step of the metabolic pathway.

It should also be understood that within the _Streptomyces_ cloning vehicles useful with the expression systems of this invention a large number of variations are possible.

For example, a wide variety of intact replicons may be employed in these cloning vectors. Such replicons may be selected from any of the plasmids and phages available in _Streptomyces_. This is a very large number because more than 30% of the _Streptomyces_ strains examined to date have plasmids. See, e.g., K. F. Chater, _supra_; European patent application 77,649; T. Kieser et al., _supra_; and C. J. Thompson et al., _Nature_, _supra_. The replicons from any of these plasmids or phages may be employed in the _Streptomyces_ cloning vehicles of this invention.

Of course, not all of the above-described replicons function with equal efficiency in a particular streptomycete. For example, some replicons may be repressed more than others in a particular streptomycete. More importantly, some replicons provide a higher copy number than other replicons. Those of skill in the art can select a replicon useful for a particular streptomycete using the principles and teachings of this invention. We prefer to use the high copy replicon of pIJ101 [T. Kieser et al., supra] in the Streptomyces cloning vectors and expression control systems of this invention.

A wide variety of selectable markers and endonuclease recognition sites may also be employed in Streptomyces cloning vehicles useful in the expression systems of this invention. For example, such markers may include resistance or sensitivity to neomycin, thiostrepton and erythromycin, or the production or lack of production of a detectable product, such as tyrosinase which polymerizes tyrosine to melanine -- a black product. In one preferred embodiment of this invention, we prefer to use tyrosinase production and lack of production as the screenable (black-white) marker for insertion of the foreign DNA sequence. In that preferred embodiment we also prefer to employ Streptomyces cloning vehicles carrying the DNA sequence encoding thiostrepton resistance.

Useful restriction sites in the Streptomyces cloning vehicles and expression systems of our invention include, for example, EcoRI, BamHI, PstI, NcoI, ClaI, SstI and any of the many other sites available or combinations of them. The selection of a particular restriction site or combination of sites is controlled by the particular Streptomyces expression control sequence and Streptomyces cloning vector employed and the particular DNA sequence desired to be expressed. For example, if a particular protein, e.g., a mature protein, is to be produced without any extraneous amino acid, the restriction site chosen must enable the linking of the DNA sequence encoding that protein to the expression control sequence in a manner that allows the desired protein to be produced by the cell.

The restriction sites useful in the products and methods of this invention may be constructed in the expression systems of this invention by methods well known in the art without departing from the scope of this invention.  Such methods include using synthetic or naturally occurring DNA segments that carry the desired endonuclease recognition site or sites or by modifying such DNA to produce the desired site or sites.

As we have described previously, the Streptomyces expression control sequences that are useful in the expression systems and methods of this invention are characterized by at least one promoter selected from the group consisting of the promoters of Streptomyces aminoglycoside phosphotransferases. It should, however, also be understood that these expression control sequences also include the other nucleotide sequences known to be necessary to allow the expression control sequence to control and to regulate the expression of a DNA sequence operatively-linked to it.  Such other necessary sequences include, for example, operator sequences, Shine-Dalgarno sequences and ribosome binding sites.

Although in the preferred embodiment of our invention, we prefer to employ the promoter and the other necessary expression sequences from the same Streptomyces aminoglycoside phosphotransferase, our invention is not so limited.  Instead, we can also combine at least one of our defined class of promoters of Streptomyces aminoglycoside phosphotransferases with other expression sequences derived from a wide variety of sources, both synthetic and natural, to construct the expression control sequence of our invention.  For example, the ribosome binding site, Shine-Dalgano sequence, or both, of the expression control sequences of this invention may be derived from the DNA sequences that control the synthesis of another Streptomyces-produced protein, from DNA sequences that control the synthesis of the protein desired to be expressed, from DNA sequences that control the synthesis of unrelated proteins, by synthesis or by any combination of these sources. All that is required for the practice of this invention is that the promoter be selected from our defined class of promoters.

It should also be understood that the Streptomyces expression control sequences of this invention may be either constitutive or controllable. However, we prefer expression control sequences that are controllable or inducible, for example, by change in temperature or by the addition of an activating compound to the fermentation culture. In that way, the culture may be grown to a high cell density before expression of the desired DNA sequence is activated. This mode of inducible operation is especially important when the product produced is inherently toxic to the cell or is toxic to the cell at the levels produced during the fermentation.

Again, it should be understood that not all Streptomyces expression control sequences will function with equal facility in controlling and regulating the expression of a particular foreign DNA sequence in a particular Streptomyces host. Instead, such factors as the transcription and translation strength of the promoter in a given host and its repression, the spatial and sequence relationships between the expression control sequence and the foreign DNA sequence to be expressed, the stability of the mRNA transcript in the host, the toxicity of the produced product to the host and the growth conditions of the host must be considered. However, by considering those factors those of skill in the art may select an appropriate combination of a <u>Streptomyces</u> expression control sequence, a foreign DNA sequence and a <u>Streptomyces</u> host to obtain a high level of expression of the desired DNA sequence.

In the preferred embodiment of this invention, we prefer to employ the <u>Streptomyces</u> expression control sequence of an aminoglycoside phosphotransferase and a <u>Streptomyces</u> host that does not produce any aminoglycoside. More preferably, the <u>Streptomyces</u> host also does not produce any restriction endonuclease that would degrade or destroy the expression system used to transform that host.

In one preferred embodiment of the expression systems and methods of our invention, we also employ a DNA sequence encoding at least a part of the signal sequence of a polypeptide or protein normally secreted from the <u>Streptomyces</u> host employed with the expression system or a part of the DNA sequence encoding the signal sequence of the foreign protein itself, if that is functional in <u>Streptomyces</u>, in order to cause the product of the inserted DNA sequence to be excreted from the <u>Streptomyces</u> host and matured. In this preferred embodiment, the DNA encoding the signal sequence is operatively linked to the <u>Streptomyces</u> expression control sequence of this invention and also linked in the same reading frame to the foreign DNA sequence desired to be expressed through the position of normal signal sequence cleavage. In this way, the product coded for by the foreign DNA sequence will be made as a putative fusion protein, consisting of the <u>Streptomyces</u> or other signal sequence and the foreign protein. The signal sequence will then effect transport of the fusion protein through the <u>Streptomyces</u> cell wall and maturation of the desired protein by cleavage of that signal sequence. See, e.g., L. Villa-Komaroff et al., "A Bacterial Clone Synthesizing Proinsulin", Proc. Natl. Acad. Sci. USA, 75, pp. 3727-31 (1981); K. Talmadge et al., "Bacteria Mature Preproinsulin To Proinsulin", Proc. Natl. Acad. Sci. USA, 77, pp. 3988-92 (1980); K. Talmadge et al., "Eukaryotic Signal Sequence Transports Insulin Antigen In Escherichia coli", Proc. Natl. Acad. Sci. USA, 77, pp. 3396-73 (1980).

In another embodiment of this invention, we employ a DNA sequence encoding at least a part of another protein or polypeptide or at least a part of the signal sequence of a protein or polypeptide that is secreted from Streptomyces and a part of the coding sequence for that protein or polypeptide between the expression control sequence of this invention and the DNA sequence encoding the desired foreign DNA product. In this embodiment a fusion protein consisting of the part the protein or polypeptide coded for by the DNA sequence and the foreign protein or polypeptide is produced. Such fusion proteins may be used as improved immunogens in vaccines and also as degradation protective devices. Such constructions may also be advantageous in improving the level of expression in a particular host.

In order that this invention may be more clearly understood, we have included the following examples for illustrative purposes only.

### EXAMPLE

### THE EXPRESSION OF A DNA SEQUENCE ENCODING BOVINE GROWTH HORMONE IN STREPTOMYCES

This example illustrates a Streptomyces expression system of this invention that is characterized by the promoter of neomycin phosphotransferase and the use of that system and promoter to express a DNA sequence encoding bovine growth hormone (BGH) in Streptomyces.

(a)     Preparation Of The Promoter
        Of Neomycin Phosphotransferase

Referring now to Figures 1 and 2, we have depicted therein in schematic outline one embodiment of the construction of an expression system of this invention.  In this system, we have employed the promoter and other expression control sequences of neomycin phosphotransferase.

In the embodiment depicted in Figures 1 and 2, we began our construction with plasmid pIJ2 [C. J. Thompson et al., Nature, 286, pp. 525-27 (1980)].  As shown in Figure 1, pIJ2 contains a genomic DNA sequence from S. fradiae which encodes neomycin phosphotransferase.  When pIJ2 was employed to transform S. livadans 66 [N. Lomovskaya et al., J. Virol., 9, pp. 258-62 (1972); John Innes Stock No. 1326], which does not produce any aminoglycoside and which is sensitive to neomycin, the transformed strain became resistant to neomycin.  Moreover, this neomycin phosphotransferase activity was expressed at a high level (2.6 U/mg) and an abundant, insert specific, 32,000 dalton protein was observed by SDS-PAGE.

We prepared mycelial soluble protein extracts of early stationary phase S. lividans (pIJ2), grown in yeast extract-malt extract medium (YEME) by sonication and centrifugation [C. J. Thompson et al., J. Bact., 151, pp. 678-85 (1982)].  We then purified the neomycin phosphotransferase activity from the extracts by ammonium sulfate precipitation (60-90% saturation) and HPLC (Waters, Brownlee column (0.046 cm), packed with a RP300 C18 matrix for reverse phase chromatography).  We eluted the neomycin phosphotransferase activity as a single peak (90% recovery) at 91% in a 40-100% acetonitrile gradient, containing 0.1% trifluoroacetic acid.  After we pooled the active fractions, we lyophilized them, dialyzed them against

water and subjected them to SDS-PAGE or amino acid analysis (Applied Biosystems, model 470A, gas phase protein sequencer). As a result of this analysis, which was done under conditions that would not have detected blocked (formylated) amino termini, we determined the amino terminal sequences of the neomycin phosphotransferase activity to be: $NH_2$-unknown-Asp-Asp-Ser-Thr-Leu-unknown-unknown-Lys-Tyr.

We then determined the nucleotide sequence of a portion of the S.fradiae-derived sequence of pIJ2 that carried the gene coding for neomycin phosphotransferase and its expression control region using the Maxam-Gilbert technique [A. Maxam & W. Gilbert, in Methods in Enzymology, L. Grosman & K. Moldave eds., Academic Press, New York, 65(1), pp. 499-560 (1980)].* We have depicted this sequence in Figures 3 and 4. We have also depicted in Figures 3 and 4, the start of the DNA sequence coding for neomycin phosphotransfrase by comparison with the amino-terminal sequence we had previously determined for this protein and the complete amino acid sequence of that protein. We have also depicted in Figures 3 and 4, parts of the 3' and 5' non-coding regions of neomycin phosphotransferase. As shown in Figure 3, there was an NcoI endonuclease recognition site at the start of the coding region of the neomycin phosphotransferase DNA sequence.

We were unable to identify specifically those DNA sequences in the 5' non-coding region depicted in Figure 3 which control the transcription and translation of the S.fradiae neomycin phosphotransferase gene by comparison with the consensus

---

*	The nucleotide sequencing was done by subcloning parts of the S. fradiae-derived DNA sequence into E. coli cloning vectors because it is simpler to produce the large amounts of DNA needed for sequencing in E. coli hosts.

sequences proposed for gene expression in E.coli [M. Rosenberg & D. Court, Ann. Rev. Genet., 13, pp. 319-53 (1979); L. Gold et al., Ann. Rev. Microbiol., 35, pp. 365-403 (1981)] or those sequences proposed for Bacillus subtilis [M. Z. Gilman et al., Nucleic Acids Res., 9, pp. 5991-6000 (1981)]. However, the presence of the streptomycete promoter in this sequence between the SstII restriction site and the start of the coding sequence (Figure 3) was confirmed by the expression of the neomycin phosphotransferase gene in Streptomyces strains transformed with various plasmids characterized by the SstII fragment of our genomic sequence.

We then subcloned a 1.1 Kb PstI fragment of pIJ2 into the PstI site of pBR322 to produce pIJ32 (Figure 1). Plasmid pIJ32 contained the coding region for the first 2/3 of neomycin phosphotransferase, as well as its promoter and the other sequences necessary for its expression. Because pBR322 carries an E. coli replicon, this subcloning allowed us to work in E. coli for our further constructions.

(b) Manipulations Of The Promoter Of Neomycin Phosphotransferase

We next isolated the 1.1 Kb PstI fragment of pIJ32 carrying the neomycin phosphotransferase-derived DNA sequences and subcloned it into the PstI site pUC9 using E.coli JM 101 [J. Vieira and J. Messing, "The pUC Plasmids, An M13mp7-Derived System For Insertion Mutagenesis And Sequencing With Synthetic Universal Primers", Gene, 19, pp. 259-68 (1982)]. We designated the resulting plasmid pEC19.

As shown in Figure 1, this plasmid had a series of useful restriction sites upstream of the promoter and expression control region of the neomycin phosphotransferase-derived DNA sequence (i.e. 5' to the gene). Again, we were able to work in E. coli hosts to manipulate this pUC9-derived plasmid.

We next isolated from pEC19 the about 600 bp NcoI-EcoRI fragment that carried the promoter and expression control region of the neomycin phosphotransferase sequence (Figure 3). Because the ATG start codon of the coding sequence of neomycin phosphotransferase was part of an NcoI site, this 600 bp fragment included the non-coding 5' region of the neomycin phosphotransferase-derived DNA sequence and ended at the beginning of the coding region for that protein.

        (c)    Combination Of The Promoter Of
               Neomycin Phosphotransferase And
               A DNA Sequence Encoding Bovine
               Growth Hormone

We combined the above-described 600 bp fragment carrying the neomycin phosphotransferase expression system with the NcoI-EcoRI fragment of p167 (Figure 1). This latter fragment contained the coding region for bovine growth hormone ("BGH").*

We designated our new plasmid pEC24 (Figure 1). It carried the promoter and expression control sequences of neomycin phosphotransferase connected in a perfect construct (via the NcoI ligation) to the ATG start codon of the coding sequence of f-met BGH.

        (d)    Expression Of f-Met Bovine Growth
               Hormone In Streptomyces Under The
               Control Of A Neomycin Phosphotransferase Expression System

We cloned the about 1300 bp BamHI fragment from pEC24 into the BglII site of Streptomyces plasmid

---

*    p167 was a gift of G. Buell. It was deposited in the American Type Culture Collection on August 16, 1982, under ATCC accession number 39173. This culture will be made available at the same time and under the same conditions as the cultures described on page 21, hereof.

pIJ702 (Figure 2). We selected the correct transformant by physical sizing and the absence of a BglII site (the ligation of a BamHI and a BglII site does not regenerate either site).

Plasmid pIJ702 is a derivative of pIJ101 [T. Kieser et al., Mol. Gen. Genet., 185, pp. 223-38 (1982)].* This plasmid contains the gene coding for thiostrepton resistance. It also contains the gene coding for tyrosinase (Figure 2). Tyrosinase polymerizes tyrosine to melanine which is black. Clones having the tyrosinase gene inactivated by a foreign DNA insertion at the BglII site of that gene will be white in the presence of tyrosine, as compared to clones without such an insert that are black. We used this black-white screening to determine which of our clones had been correctly transformed with the DNA fragment containing the Streptomyces neomycin phosphotransferase promoter and expression control sequences and the coding sequence of BGH.

We transformed S.lividans 66 [John Innes Stock No. 1326; Lomovskaya et al. J. Virol., 9, pp. 258-62 (1972)] with plasmid pIJ702 and selected the white colonies. We designated one of these colonies, containing plasmid pBG22, S. lividans TC98 (Figure 2). A sample of the culture was deposited (infra).

We cultured S. lividans TC98 in YEME and also in minimal media for various times up to several weeks at 30°C. We then prepared cell extracts by centrifugation and lysozyme-treatment or sonication and assayed the extracts for the presence of BGH.

---

* We have deposited a sample of pIJ703, another derivative of pIJ101 (infra). Plasmids pIJ702 and pIJ703 are identical hybrids except that the fragments containing the gene coding the for the tyrosinase determinant (mel) and the gene coding for thiostrepton resistance (tsr) are transposed [E. Katz et al., J. Gen. Microbiol., 129, pp. 2703-14 (1983)].

We detected BGH in our extracts. The BGH production of our transformed Streptomyces strains initially rose to a steady state level. That level of production was then maintained for several weeks after vegetative growth of the Streptomyces had been completed.

We analyzed the proteins in the S. lividans lysates by SDS-PAGE followed by immuno-blotting and observed no immunoreacative products smaller than the actual hormone, which suggests that BGH is not degraded in S. lividans (although our procedure might not detect breakdown products smaller than 14 kd).

We introduced plasmids containing the BGH gene into E. coli and S. lividans to compare expression of the gene in the two hosts. When the yields were nomalized to the total protein content of the cells, we determined that S. lividans contained three times the hormone contained in E. coli. No BGH was detected in the culture medium following growth of either strain.

We found that we could appreciably increase the yield of BGH from S. lividans by replacing the DNA sequence coding for mature BGH with a DNA insert in which the coding region for the first nine amino acids of mature BGH had been removed, and the coding region for the tenth amino acid modified from CTG to TTG, a change that does not alter its amino acid (leucine).

It should, of course, be understood that while the above illustrative embodiment of this invention involved the combination of the promoter and other expression control sequences of neomycin phosphotransferase with a DNA sequence encoding BGH, the construction could as well have been effected by various other sequences of steps. For example, the DNA sequence encoding BGH could have been in-

B.0217

serted into a <u>Streptomyces</u> cloning vector already carrying the desired expression control sequence or the expression control sequence could have been inserted into a <u>Streptomyces</u> cloning vector already carrying the BGH coding sequence.

It should also be understood that the entire expression control sequence in our Example need not have been taken from the neomycin phosphotransferase gene. As described previously, only the promoter of the expression control sequence of this invention need to be derived from a <u>Streptomyces</u> aminoglycoside phosphotransferase. The other elements of the expression control sequence may be independently derived or constructed.

It should also be understood that the manipulations leading to the final <u>Streptomyces</u> expression system need not have been done in <u>E. coli</u>. Rather, they could have been done in any host strain. However, we prefer to employ <u>E. coli</u> because of the ease of manipulation, transformation and growth in that strain.

Finally, it should be understood that any <u>Streptomyces</u> host in which the replicon of pIJ702 is functional could have been used in our illustrative example to produce bovine growth hormone.

Microorganisms and recombinant DNA molecules prepared by the processes of this invention are exemplified by cultures deposited in the American Type Culture Collection, Rockville, Maryland on June 6, 1983, and identified there as:

A: <u>Streptomyces lividans</u> 66 (pIJ703)
B: <u>Streptomyces lividans</u> 66 (pBG22) or <u>S. lividans</u> TC 98

These cultures were assigned accession numbers 39378 and 39379, respectively.

A process similar to that described above has also been used successfully to express a gene that encodes human serum albumin in <u>S. Lividans</u>. Thus, illustrating that the methods of this invention are useful for the expression of a variety of genes in <u>Strepomyces</u>.

While we have hereinbefore presented a number of embodiments of this invention, it is apparent that our basic construction can be altered to provide other embodiments which utilize the processes and compositions of this invention. Therefore, it will be appreciated that the scope of this invention is to be defined by the claims appended hereto, rather than by the specific embodiments which have been presented hereinbefore by way of example.

0148552

We Claim:

1. A <u>Streptomyces</u> expression system for expressing foreign DNA sequences in <u>Streptomyces</u> characterized by at least one promoter selected from the group consisting of the promoters of <u>Streptomyces</u> aminoglycoside phosphotransferases.

2. The expression system of claim 1, wherein said <u>Streptomyces</u> aminoglycoside phosphotransferase is neomycin phosphotransferase.

3. The expression system of claim 1 or 2, wherein said foreign DNA sequence is selected from the group consisting of eukaryotic and viral DNA sequences.

4. The expression system of claim 3, wherein said foreign DNA sequence is selected from the group consisting of DNA sequences encoding the human and animal leukocyte, fibroblast and immune interferons, human insulin, animal and human growth and other hormones, human blood factors, hepatitis B viral antigens and FMD viral antigens.

5. A method of expressing a foreign DNA sequence in <u>Streptomyces</u> characterized by the step of operatively linking said foreign DNA sequence to a <u>Streptomyces</u> expression control sequence, said expression control sequence being characterized by at least one promoter selected from the group consisting of the promoters of <u>Streptomyces</u> aminoglycoside phosphotransferases.

6. The method of claim 5, wherein said <u>Streptomyces</u> aminoglycoside phosphotransferase is neomycin phosphotransferase.

7. The method of claim 5 or 6, wherein said foreign DNA sequence is selected from the group consisting of eukaryotic and viral DNA sequences.

8. The method of any one of claims 5 to 7, wherein said foreign DNA sequence is operatively linked to said <u>Streptomyces</u> expression control se-

quence through a DNA sequence encoding at least a part of the signal sequence of a protein or polypeptide secreted from <u>Streptomyces</u> or at least part of another signal sequence functional in <u>Streptomyces</u>, said DNA sequence encoding the signal sequence being expressed together with said foreign DNA sequence under the control of said <u>Streptomyces</u> expression control sequence to afford excretion and maturation of the protein or polypeptide coded for by the foreign DNA sequence.

9. The method of any one of claims 5 to 7, wherein said foreign DNA sequence is operatively linked to said <u>Streptomyces</u> expression control sequence through a DNA sequence encoding a part of another protein or polypeptide or a part of the signal sequence and coding sequence of another protein or polypeptide, said DNA sequence being expressed together with said foreign DNA sequence under the control of said <u>Streptomyces</u> expression control sequence to produce a fused protein or polypeptide.

10. A method of amplifying the production of a <u>Streptomyces</u>-derived antibiotic or other product characterized by the step of employing at least one promoter selected from the group consisting of the promoters of <u>Streptomyces</u> aminoglycoside phosphotransferases.

11. The method of claim 10, wherein said aminoglycoside phosphotransferase is neomycin phosphotransferase.

12. The method of claim 10, wherein said aminoglycoside phosphotransferase is kanamycin phosphotransferase.

FIG.1

1/5

0148552

# FIG.2

EcoRI
Bam HI
Sal I
Pst I
pEC 24
Nco I
BGH
Bam HI

(1) Bam HI
(2) ← Bgl II ←

Tyrosinase
pIJ 702
Bgl II
Thiostrepton

Tyrosinase
Sal I
Pst I
Nco I
BGH
Thiostrepton
Tyrosinase

(1) S. lividans 66

S. lividans 66 (pB 622)

FIGURE 3

```
    Nar  1
CGC GGC GCC CGC GCC GCC AGC TCG GCG GGG CGG ACC CGG ACC CGG CCG CCG AGG TCC TCG      60


CCG CCG ACC GGG AGG CGT CGG CCT CGC CGC CGA AGA CCG CCG TCC TGC TGC GGC TCA CGG     120

    Rsa 1
AGG CGT ACC TCT CGC CCT GCG CGC GGG CCT TCG ACC CCG CCG GGA CCT CCG GCA CCG GGC     180

  Sst 2                                           Nar 1
CCG CGG GCG ACG CCG GGC GCA CCG GGT CCA CCG GCG CCC CCC CAC CCC GCA CAG AAT GTC     240


CGA AAC CCC TAC GGG CCC CGA CGA AAG GCG CGG AAC GGC GTC TCC GCC TCT GCC ATG ATG     300

    Nco I                                 Rsa 1
CCG CCC ATG GAC GAC AGC ACG TTG CGC CGG AAG TAC CCG CAC CAC GAG TGG CAC GCA GTG     360
        Met Asp Asp Ser Thr Leu Arg Arg Lys Tyr Pro His His Glu Trp His Ala Val

          Hinf 1   Nar 1
AAC GAA GGA GAC TCG GGC GCC TTC GTC TAC CAG CTC ACC GGC GGC CCC GAG CCC CAG CCC     420
Asn Glu Gly Asp Ser Gly Ala Phe Val Tyr Gln Leu Thr Gly Gly Pro Glu Pro Gln Pro

  Sst 1
GAG CTC TAC GCG AAG ATC GCC CCC CGC GCC CCC GAG AAC TCC GCC TTC GAC CTG TCC GGC     480
Glu Leu Tyr Ala Lys Ile Ala Pro Arg Ala Pro Glu Asn Ser Ala Phe Asp Leu Ser Gly
```

FIGURE 3A

```
                                                     Bam H 1
GAG GCC GAC CGG CTG GAG TGG CTC CAC CGC CAC GGG ATC CCC GTC CCC CGC GTC GTC GAG      540
Glu Ala Asp Arg Leu Glu Trp Leu His Arg His Gly Ile Pro Val Pro Arg Val Val Glu

CGC GGT GCC GAC GAC ACC GCC GCG TGG CTC GTC ACG GAG GCC GTC CCC GGC GTC GCG GCG      600
Arg Gly Ala Asp Asp Thr Ala Ala Trp Leu Val Thr Glu Ala Val Pro Gly Val Ala Ala

GCC GAG GAG TGG CCC GAG CAC CAG CGG TTC GCC GTG GTC GAG GCG ATG GCG GAG CTG GCC      660
Ala Glu Glu Trp Pro Glu His Gln Arg Phe Ala Val Val Glu Ala Met Ala Glu Leu Ala

                                                          Nar 1
CGC GCC CTC CAC GAG CTG CCC GTG GAG GAC TGC CCC TCC GAC CGG CGC CTC GAC GCG GCG      720
Arg Ala Leu His Glu Leu Pro Val Glu Asp Cys Pro Ser Asp Arg Arg Leu Asp Ala Ala

                                                              Pst 1
GTC GCC GAG GCC CGG CGG AAC GTC GCC GAG GGC TTG GTG GAC CTC GAC GAC CTG CAG GAG      780
Val Ala Glu Ala Arg Arg Asn Val Ala Glu Gly Leu Val Asp Leu Asp Asp Leu Gln Glu

                                      Sst 1
GAG CGG GCC GGG TGG ACC GGC GAC CAG CTC CTG GCG GAG CTC GAC CGC ACC CGT CCC GAG      840
Glu Arg Ala Gly Trp Thr Gly Asp Gln Leu Leu Ala Glu Leu Asp Arg Thr Arg Pro Glu
```

4/5

0148552

FIGURE 4

```
AAG GAG GAC CTG GTC GTC TGC CAT GGC GAC CTG TGC CCC AAC AAC GTC CTG CTC GAC CCC     900
Lys Glu Asp Leu Val Val Cys His Gly Asp Leu Cys Pro Asn Asn Val Leu Leu Asp Pro

              Bst E2
GGG ACC TGC CGG GTC ACC GGC GTG ATC GAC GTC GGC CGC CTC GGG GTC GCC GAC CGC CAC     960
Gly Thr Cys Arg Val Thr Gly Val Ile Asp Val Gly Arg Leu Gly Val Ala Asp Arg His

GCC GAC ATC GCC TTG GCC GCC CGC GAG CTG GAG ATC GAC GAG GAC CCC TGG TTC GGC CCC    1020
Ala Asp Ile Ala Leu Ala Ala Arg Glu Leu Glu Ile Asp Glu Asp Pro Trp Phe Gly Pro

                                   Rsa 1 Nar 1        Sal 1
GCC TAC GCC GAG CGG TTC CTG GAG CGG TAC GGC GCC CAC CGC GTC GAC AAG GAG AAG CTG    1080
Ala Tyr Ala Glu Arg Phe Leu Glu Arg Tyr Gly Ala His Arg Val Asp Lys Glu Lys Leu

                                       Xba 1
GCC TTC TAC CAG CTT CTC GAC GAG TTC TTC TAG AGC CGC CCC GCA GGG CGC TCC GCA GGC    1140
Ala Phe Tyr Gln Leu Leu Asp Glu Phe Phe***

CGC TTC CGG ACC ACT CCG GAA GCG GCC GTG CGG TCG GAG GUA CCC GGC CGC CTT GGA GAC    1200

    Nar 1              Sst 2
CGG CGC CCG GCC CCC GCT TTC CGC GGC UTG GCC GGA GCC GTC AGA GGC CGT GGT ACG GGT    1260

        Kpn 1
TGG CGG CGA GGT ACC GGG CT                                                          1280
```

# DECLARATION PURSUANT TO RULE 28, PARAGRAPH 4,
# OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

**Accession numbers of the deposits:**

ATCC 39378 and 39379.

0148552

**European Patent Office**

**EUROPEAN SEARCH REPORT**

Application number

| | DOCUMENTS CONSIDERED TO BE RELEVANT | | EP 84303755.7 |

| Category | Citation of document with indication, where appropriate, of relevant passages | Relevant to claim | CLASSIFICATION OF THE APPLICATION (Int. Cl. ³) = |
|---|---|---|---|
| D,X | NATURE, vol. 286, no. 5772, July 31, 1980 (London, New York) C.J. THOMPSON et al. "DNA cloning in Streptomyces: resistance genes from antibiotic-producing species" pages 525-527 * Page 525, right column, lines 1-33 * -- | 1,2,5, 6,10,11 | C 12 N 15/00// C 12 R 1/465 |
| D,X | GENE, vol. 20, 1982 (Amsterdam) C.J. THOMPSON et al.: "Physical analysis of antibiotic-resistance genes from Streptomyces and their use in vector construction" pages 51-62 * Page 51, Summary * -- | 1,2,5, 6,10,11 | |
| D,X | JOURNAL OF BACTERIOLOGY, vol. 151, no. 2, August 1982, (Washington D.C.) C.J. THOMPSON et al.: "Biochemical Characterization of Resistance Determinants Cloned from Antibiotic- -Producing Streptomycetes" pages 678-685 * Pages 678, Summary * -- | 1,2,5, 6,10,11 | **TECHNICAL FIELDS SEARCHED (Int. Cl. ³)** C 12 N |
| A | EP - A2 - 0 077 650 (ELI LILLY AND COMPANY) * Claims 1,2,12,15 * ---- | 1,2,5, 6,10,11 | |

The present search report has been drawn up for all claims

| Place of search | Date of completion of the search | Examiner |
|---|---|---|
| VIENNA | 29-08-1984 | WOLF |